# EUROPEAN PATENT APPLICATION

(11) **EP 3 363 468 A1**
(43) Date of publication of application: **22.08.2018**
(21) Application number: 16855204.0
(22) Date of filing: 08.09.2016
(51) Int. Cl.: A61L 2/10, A61L 9/20, C02F 1/32

(54) **FLUID STERILIZATION DEVICE AND FLUID STERILIZATION METHOD**

(30) Priority: 13.10.2015 JP 2015202206
(71) Applicant: Nikkiso Co., Ltd., Shibuya-ku, Tokyo 150-6022 (JP)
(72) Inventor: OCHI Tetsumi, Hakusan-shi Ishikawa 924-0004 (JP); KONAGAYOSHI Hidenori, Hakusan-shi Ishikawa 924-0004 (JP); TORII Nobuhiro, Hakusan-shi Ishikawa 924-0004 (JP); KIUCHI Hiroki, Hakusan-shi Ishikawa 924-0004 (JP)
(74) Representative: Copsey, Timothy Graham
(86) International application number: PCT/JP2016/076422
(87) International publication number: WO 2017/064950

(57) **Abstract**

A fluid sterilization device 10 includes a straight pipe 20 that constitutes a flow passage 12 extending in a longitudinal direction, and a light source 40 that emits ultraviolet light in the longitudinal direction toward a fluid flowing in a laminar state within the flow passage 12. The light source 40 includes a light emitting element 42 for emitting ultraviolet light and emits ultraviolet light so as to provide an intensity distribution on a cross section of the flow passage perpendicular to a longitudinal direction, in which the ultraviolet intensity in an area near the center is higher than the ultraviolet intensity around the area.

## Description

### [TECHNICAL FIELD]

The present invention relates to a fluid sterilization device and a fluid sterilization method and, more particularly, to a technology of sterilizing a fluid by irradiating the fluid with ultraviolet light.

### [BACKGROUND ART]

It is known that ultraviolet light has sterilization capability. Devices that radiate ultraviolet light are used for sterilization in medical and food processing fronts. Devices that sterilize a fluid such as water continuously by irradiating the fluid with ultraviolet light are also used. One example is a device in which an ultraviolet LED is provided on the inner wall at a pipe end of a flow passage formed by a straight metal pipe (see Patent Document 1, for example).

### [PRIOR ART REFERENCE]

### [PATENT DOCUMENT]

[Patent Document 1] Japanese Unexamined Patent Application Publication No. 2011-16074

### [DISCLOSURE OF INVENTION]

### [PROBLEM(S) TO BE SOLVED BY THE INVENTION]

In order to irradiate a fluid with ultraviolet light with high efficiency, it is desirable to control the state of flow of the fluid in the flow passage appropriately and to radiate ultraviolet light in a mode suitable to the state of flow.

The present invention has been made in view of such a problem, and an illustrative purpose thereof is to provide a fluid sterilization device with improved irradiation efficiency of ultraviolet light for a fluid flowing within a flow passage.

### [MEANS TO SOLVE THE PROBLEM(S)]

A fluid sterilization device of one embodiment of the present invention includes: a straight pipe that constitutes a flow passage extending in a longitudinal direction; and a light source that emits ultraviolet light in the longitudinal direction toward a fluid flowing in a laminar state within the flow passage. The light source includes a light emitting element for emitting ultraviolet light and emits ultraviolet light so as to provide an intensity distribution on a cross section of the flow passage perpendicular to a longitudinal direction, in which the ultraviolet intensity in an area near the center is higher than the ultraviolet intensity around the area.

According to this embodiment, since a fluid flowing in a laminar state is irradiated with ultraviolet light, the sterilization effect can be improved compared to the case where a fluid in a turbulent state is irradiated with ultraviolet light. Based on the inventors' findings, it is found that, when sterilization treatment is performed by irradiating a fluid within a flow passage of a straight pipe with ultraviolet light, in the case of a laminar state, about seven times the sterilization effect of the case of a turbulent state can be obtained. Also, a fluid in a laminar state has a velocity distribution in which the flow velocity near the center is higher, and the flow velocity near the inner wall of the pipe is lower; accordingly, by increasing the ultraviolet intensity near the center according to the velocity distribution, the fluid flowing within the flow passage can be effectively irradiated with ultraviolet light, so that the sterilization effect can be improved.

The straight pipe may include a first end part provided with an inlet port through which a fluid flows into the straight pipe in the longitudinal direction, and also include a second end part located opposite to the first end part. The light source may be disposed at the second end part.

The light source and the inlet port may be disposed on the central axis of the straight pipe.

The straight pipe may include an outlet port provided on the second end part, through which a fluid flows out in a direction intersecting the longitudinal direction.

The straight pipe may include an outlet port provided on the second end part, through which a fluid flows out in the longitudinal direction. The light source may include a plurality of light emitting elements disposed so as to surround the outlet port. The plurality of light emitting elements may emit ultraviolet light in the longitudinal direction toward a fluid flowing within the flow passage.

The fluid sterilization device may further include a straightening plate that makes a flow of a fluid within the flow passage a laminar flow.

Another embodiment of the present invention relates to a fluid sterilization method. In the fluid sterilization method, a flow of a fluid in a laminar state is made within a straight pipe that constitutes a flow passage extending in a longitudinal direction, and ultraviolet light is emitted in the longitudinal direction toward a fluid flowing in a laminar state within the flow passage so as to provide an intensity distribution on a cross section of the flow passage perpendicular to the longitudinal direction, in which the ultraviolet intensity in an area near the center is higher than the ultraviolet intensity around the area.

According to this embodiment, since a fluid flowing in a laminar state is irradiated with ultraviolet light, the sterilization effect can be improved compared to the case where a fluid in a turbulent state is irradiated with ultraviolet light. Also, a fluid in a laminar state has a velocity distribution in which the flow velocity near the center is higher, and the flow velocity near the inner wall of the pipe is lower; accordingly, by increasing the ultraviolet intensity near the center according to the velocity distribution, the fluid flowing within the flow passage can be effectively irradiated with ultraviolet light, so that the sterilization effect can be improved.

### [ADVANTAGEOUS EFFECTS OF INVENTION]

The present invention enables improvement in irradiation efficiency of ultraviolet light for a fluid flowing within a flow passage, thereby also improving the sterilization capability.

### [BRIEF DESCRIPTION OF DRAWINGS]

Fig. 1 is a sectional view that schematically shows a configuration of a fluid sterilization device according to a first embodiment;
Fig. 2 is a contour diagram that shows an ultraviolet intensity distribution within a flow passage;
Fig. 3 is a contour diagram that shows a velocity distribution of a fluid in a turbulent state;
Fig. 4 is a contour diagram that shows a velocity distribution of a fluid in a laminar state;
Fig. 5 is a front view that schematically shows a configuration of a light source according to a modification;
Fig. 6 is a sectional view that schematically shows a configuration of a fluid sterilization device according to a

### second embodiment; and

Fig. 7 is a sectional view that schematically shows a configuration of the fluid sterilization device shown in Fig. 6.

### [MODE FOR CARRYING OUT THE INVENTION]

In the following, a mode for carrying out the present invention will be described in detail with reference to the drawings. Like reference characters denote like elements in the description, and the same description will be omitted as appropriate.

### (First Embodiment)

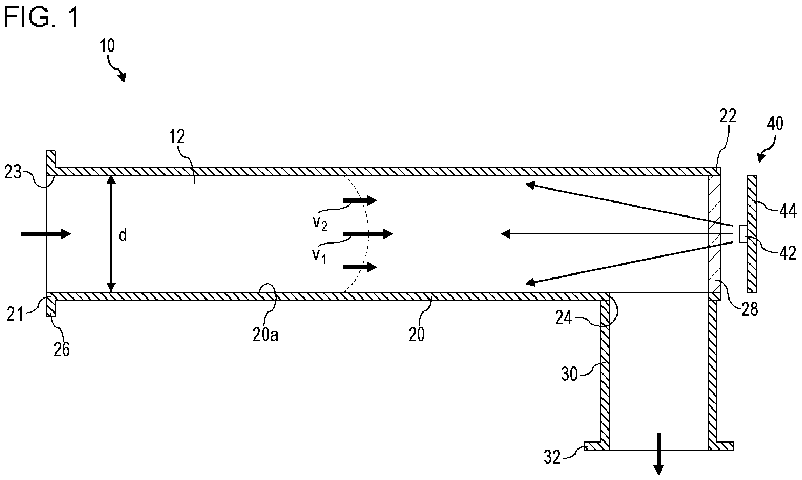

Fig. 1 is a diagram that schematically shows a configuration of a fluid sterilization device 10 according to a first embodiment. The fluid sterilization device 10 comprises a straight pipe 20, an outflow pipe 30, and a light source 40. The light source 40 is disposed at an end part (a second end part 22) of the straight pipe 20 to emit ultraviolet light toward the inside of the straight pipe 20. The fluid sterilization device 10 is used to irradiate a fluid, such as water, flowing within the straight pipe 20 with ultraviolet light for sterilization treatment.

The straight pipe 20 includes a first end part 21, the second end part 22, a first flange 26, and a window part 28. The straight pipe 20 extends in the longitudinal direction from the first end part 21 toward the second end part 22. The first end part 21 is provided with an inlet port 23 through which a fluid flows into the straight pipe 20 in the longitudinal direction of the straight pipe 20, and with the first flange 26 for connecting the inlet port 23 to another pipe or the like. The second end part 22 is provided with the window part 28 for transmitting ultraviolet light emitted by the light source 40. The window part 28 is constituted by a member having high ultraviolet light transmittance, such as quartz (SiO₂), sapphire (Al₂O₃), and amorphous fluororesin.

On the second end part 22 is provided an outlet port 24 through which a fluid flows out in a direction intersecting or perpendicular to a longitudinal direction of the straight pipe 20. The outlet port 24 is provided on a side wall of the straight pipe 20 and, to the outlet port 24, the outflow pipe 30 is attached. One end of the outflow pipe 30 is attached to the outlet port 24, and the other end thereof is provided with a second flange 32. Accordingly, the straight pipe 20 and the outflow pipe 30 form a flow passage 12 of an L shape. A fluid flowing in through the first flange 26 will flow through the inlet port 23, straight pipe 20, outlet port 24, and outflow pipe 30 to flow out through the second flange 32.

Each of the straight pipe 20 and the outflow pipe 30 is made of a metal material or resin material. An inner wall surface 20a of the straight pipe 20 is desirably made of a material having a high ultraviolet light reflectance, such as mirror-polished aluminum (Al) and polytetrafluoroethylene (PTFE), which is fully fluorinated resin. By using such a material to configure the inner wall surface 20a of the straight pipe 20, ultraviolet light emitted by the light source 40 is reflected by the inner wall surface 20a, so as to be transmitted in the longitudinal direction of the straight pipe 20. Since PTFE is a chemically stable material having a high ultraviolet light reflectance, it is suitable as a material of the straight pipe 20 that constitutes a flow passage of the fluid sterilization device.

An inner diameter d of the straight pipe 20 and an average flow velocity v of a fluid flowing through the flow passage 12 are adjusted so that the fluid flows through the flow passage 12 in a laminar state. More specifically, the inner diameter d and the average flow velocity v are adjusted so that the Reynolds number Re in the flow passage 12 becomes the critical Reynolds number in a laminar state or lower, based on the equation of Re=v·d/ν (ν: the coefficient of kinematic viscosity of the fluid). The value of the critical Reynolds number or lower may be the Reynolds number of 3000 or lower, preferably 2500 or lower, and more preferably 2320 or lower. By allowing a fluid to flow through the inlet port 23 into the flow passage 12 in the longitudinal direction, the fluid flows in a laminar state toward the second end part 22. When a fluid is flowing in a laminar state, the flow velocity distribution is made so that a flow velocity v₁ of the fluid flowing near the central axis of the straight pipe 20 is relatively higher, and a flow velocity v₂ of the fluid flowing near the inner wall surface 20a of the straight pipe 20 is relatively lower. In an ideal laminar state, the velocity distribution of the fluid flowing through the flow passage 12 is expressed by an equation for a paraboloid of revolution.

The light source 40 includes a light emitting element 42 and a substrate 44. The light emitting element 42 is an light emitting diode (LED) that emits ultraviolet light of which the center wavelength or peak wavelength falls within a range of about 200 nm to 350 nm. The light emitting element 42 may suitably emit ultraviolet light of which the wavelength falls within a range of about 260 nm to 270 nm, which has high sterilization efficiency. As such an ultraviolet LED, one using aluminum gallium nitride (AlGaN) is known, for example.

The light emitting element 42 is an LED having a predetermined directional angle or light distribution angle and may be an LED having a wide light distribution angle of 120 degrees or greater (in total angular value), for example. As the light emitting element 42, there is an LED of a surface mount device (SMD) type having high output intensity. The light emitting element 42 is disposed on the central axis of the straight pipe 20 and mounted on the substrate 44 so as to face the window part 28. The substrate 44 is constituted by a member having high thermal conductivity, and copper (Cu) or aluminum (Al) may be used as a base material thereof, for example. Heat generated by the light emitting element 42 is dissipated through the substrate 44.

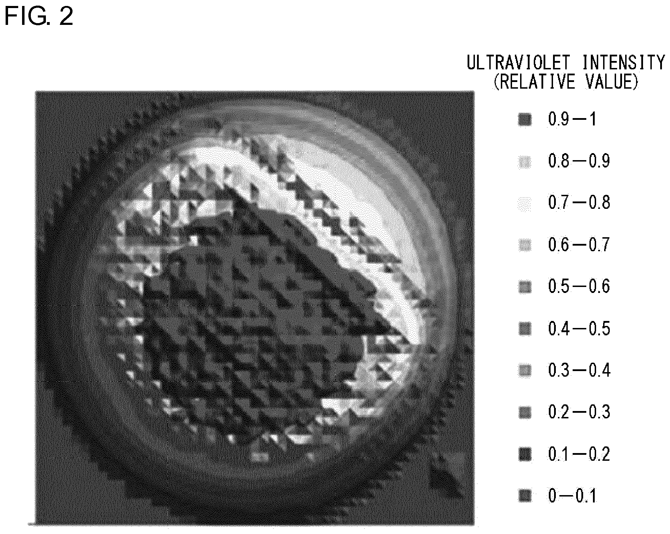

Fig. 2 is a contour diagram that shows an ultraviolet intensity distribution within the flow passage 12. Since the light emitting element 42 emits ultraviolet light with a predetermined light distribution angle, the intensity distribution is made so that the ultraviolet intensity in an area near the center is higher than the ultraviolet intensity around the area. As a result, the intensity distribution of ultraviolet light within the straight pipe 20 is made so that, on a cross section of the flow passage 12 perpendicular to the longitudinal direction, the ultraviolet intensity near the central axis is higher, whereas the ultraviolet intensity near the inner wall surface 20a is lower.

With the configuration set forth above, the fluid sterilization device 10 irradiates a fluid flowing within the straight pipe 20 with ultraviolet light so as to perform sterilization treatment on the fluid. The light source 40 emits ultraviolet light so that the intensity thereof near the center of the straight pipe 20 is higher, and the intensity thereof near the inner wall surface 20a of the straight pipe 20 is lower. Meanwhile, the fluid is made to flow through the flow passage 12 in a laminar state in which a flow velocity v₁ near the center is higher, and a flow velocity v₂ near the inner wall surface 20a is lower. As a result, the amount of ultraviolet light energy acting on the fluid flowing in a laminar state through the straight pipe 20 can be equalized, irrespective of the position in a radial direction of the flowing fluid. Accordingly, the entirety of the fluid flowing through the straight pipe 20 can be irradiated with ultraviolet light having a predetermined amount or more of energy, so that the sterilization effect on the entire fluid can be improved.

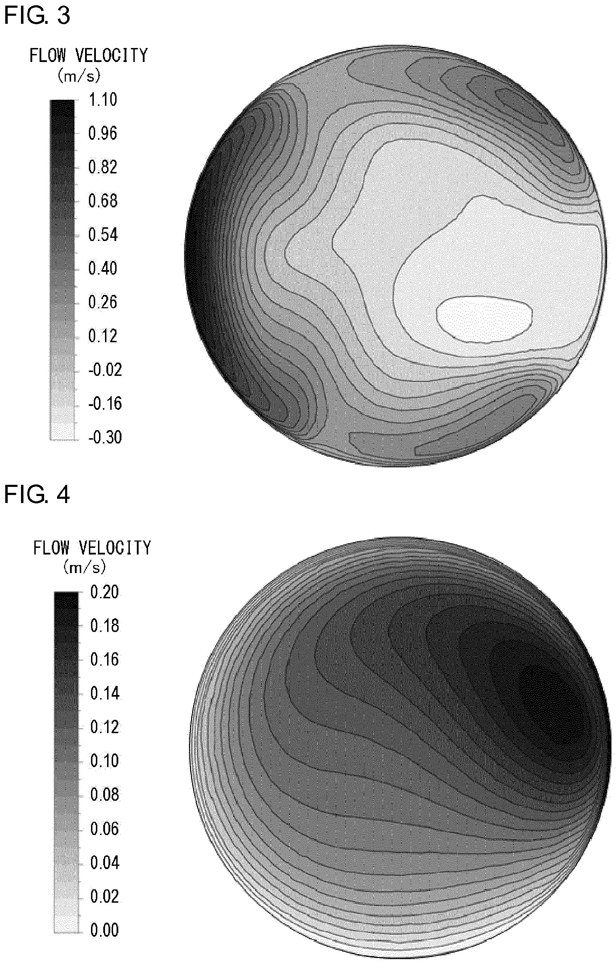

There will now be described an effect of the fluid sterilization device 10 with reference to a comparative example. Fig. 3 is a contour diagram that shows a velocity distribution of a fluid in a turbulent state when the fluid flows through the straight pipe 20 under the condition of the Reynolds number Re = 4961. The example of Fig. 3 shows a state where the flow velocity in a partial area near the inner wall surface 20a is the highest, and the flow velocity near the center shows a negative value; however, the velocity distribution of a fluid is not steady and occasionally changes with time. Under the condition of such a turbulent state, when bacterial liquid containing Escherichia coli was used as the fluid, the survival rate of the Escherichia coli in the fluid that had flowed through the sterilization device was 0.53%.

Fig. 4 is a contour diagram that shows a velocity distribution of a fluid in a laminar state when the fluid flows through the straight pipe 20 under the condition of the Reynolds number Re = 2279. In the example of Fig. 4, although the area in which the flow velocity is highest is shifted to an upper right area, the flow velocity distribution basically shows that the flow velocity near the center is higher, and the flow velocity near the inner wall surface 20a is lower. Under the condition of such a laminar state, when bacterial liquid containing Escherichia coli was used, the survival rate of the Escherichia coli in the fluid that had flowed through the sterilization device was 0.07%. From these results, it is found that, in a laminar state, about seven times the sterilization effect of the case of a turbulent state can be obtained. Thus, in the present embodiment, since a fluid in a laminar state can be irradiated with ultraviolet light of which the intensity distribution corresponds to the flow velocity distribution of a fluid in a laminar state, the sterilization effect on the fluid can be improved.

Also, in the present embodiment, since the inlet port 23 and the light source 40 are disposed on the central axis of the straight pipe 20, a smooth flow of the fluid can be made in the direction toward ultraviolet light emitted by the light source 40. Since the inlet port 23 is disposed at a position opposite to the light source 40, the fluid reaches a laminar state with less turbulence while flowing through the straight pipe 20, and the fluid in the laminar state can be irradiated with strong ultraviolet light. Accordingly, the situation can be prevented in which inconsistency occurs in the amount of ultraviolet light energy acting on the fluid because part of the fluid flows through an area of low ultraviolet intensity at a high velocity or because part of the fluid swirls and remains in an area of high ultraviolet intensity, for example, so that the influence of sterilization effect reduced by the situation can be curbed.

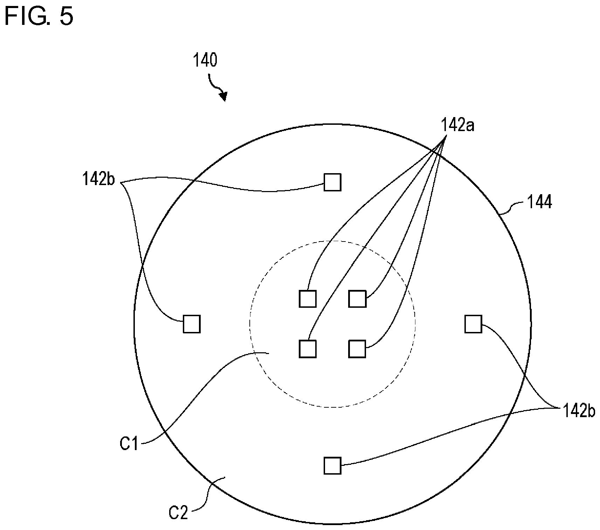

Fig. 5 is a front view that schematically shows a configuration of a light source 140 according to a modification. The light source 140 includes multiple light emitting elements 142a and 142b, and a substrate 144. The light source 140 includes multiple first light emitting elements 142a densely disposed in a central area C1 of the substrate 144, and multiple second light emitting elements 142b disposed to dot a peripheral area C2 of the substrate 144. Each of the first light emitting elements 142a and the second light emitting elements 142b is configured similarly to the light emitting element 42 described previously.

In the light source 140, since the first light emitting elements 142a are densely disposed in the central area C1, ultraviolet light having relatively high intensity is outputted from the central area C1. Meanwhile, since the second light emitting elements 142b are disposed to dot the peripheral area C2, ultraviolet light having relatively low intensity is outputted from the peripheral area C2. Accordingly, even when the diameter d of the straight pipe 20 is enlarged to increase the flow rate, by applying the light source 140 of the present modification to the fluid sterilization device 10 described previously, a fluid can be irradiated with ultraviolet light having an intensity distribution in which the ultraviolet intensity is higher near the center and lower near the inner wall surface 20a.

### (Second Embodiment)

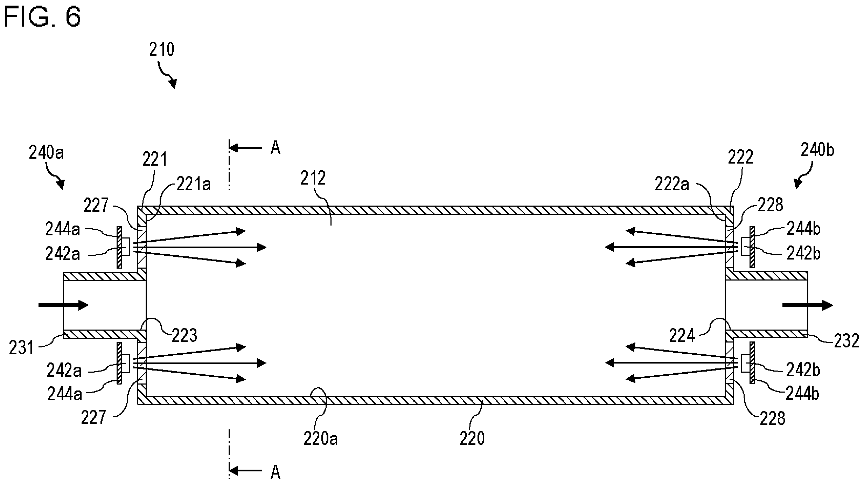

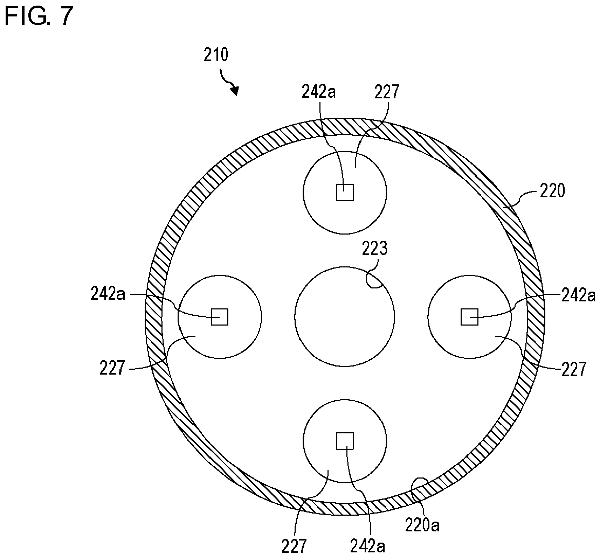

Each of Figs. 6 and 7 is a sectional view that schematically shows a configuration of a fluid sterilization device 210 according to a second embodiment, and Fig. 7 corresponds to a sectional view taken along line A-A in Fig. 6. The fluid sterilization device 210 comprises a straight pipe 220, an inflow pipe 231, an outflow pipe 232, multiple first light sources 240a, and multiple second light sources 240b. The fluid sterilization device 210 is different from the fluid sterilization device in the aforementioned first embodiment in that the inflow pipe 231 and the outflow pipe 232 are disposed on the central axis of the straight pipe 220, so that a flow passage 212 of a linear shape, instead of an L shape, is formed. In the following, the present embodiment will be described mainly for the differences from the first embodiment.

The straight pipe 220 extends from a first end part 221 toward a second end part 222. The first end part 221 is provided with a first end surface 221a perpendicular to a longitudinal direction of the straight pipe 220, and with an inlet port 223 positioned near the center of the first end surface 221a. Also, the first end surface 221a is provided with multiple first window parts 227 for transmitting ultraviolet light emitted by the first light sources 240a. To the inlet port 223, the inflow pipe 231 extending in a longitudinal direction of the straight pipe 220 is attached. Through the inflow pipe 231, a fluid flows into the straight pipe 220 in the longitudinal direction of the straight pipe 220, thereby reducing the generation of turbulence in the flow within the flow passage 212.

The second end part 222 is configured similarly to the first end part 221. The second end part 222 is provided with a second end surface 222a perpendicular to a longitudinal direction of the straight pipe 220, and with an outlet port 224 positioned near the center of the second end surface 222a. Also, the second end surface 222a is provided with multiple second window parts 228 for transmitting ultraviolet light emitted by the second light sources 240b. To the outlet port 224, the outflow pipe 232 extending in a longitudinal direction of the straight pipe 220 is attached. Through the outflow pipe 232, a fluid flows out of the straight pipe 220 in the longitudinal direction of the straight pipe 220, thereby reducing the generation of turbulence in the flow within the flow passage 212.

The first light sources 240a include multiple first light emitting elements 242a and multiple first substrates 244a. The multiple first light emitting elements 242a are disposed on all sides so as to surround the inlet port 223 and mounted respectively on the first substrates 244a, as shown in Fig. 7. Each of the multiple first light emitting elements 242a emits ultraviolet light in the longitudinal direction of the straight pipe 220 toward the inside of the straight pipe 220, through the corresponding first window part 227.

Although the example of Fig. 7 shows the case where the first light emitting elements 242a are provided at four positions, the number of positions at which the first light emitting elements 242a are provided may be three or less, or may be five or more. It is suitable that the multiple first light emitting elements 242a are evenly spaced so as to emit ultraviolet light to the entire fluid flowing within the flow passage 212. Since the multiple first light emitting elements 242a are evenly spaced so as to surround the inlet port 223, the first light sources 240a emit ultraviolet light having an intensity distribution in which the ultraviolet intensity is higher near the center of the straight pipe 220 and lower near an inner wall surface 220a of the straight pipe 220.

The second light sources 240b include multiple second light emitting elements 242b and multiple second substrates 244b and are configured similarly to the first light sources 240a. The multiple second light emitting elements 242b are disposed on all sides so as to surround the outlet port 224 and mounted respectively on the second substrates 244b. Each of the multiple second light emitting elements 242b emits ultraviolet light in the longitudinal direction of the straight pipe 220 toward the inside of the straight pipe 220, through the corresponding second window part 228. As with the first light sources 240a, the second light sources 240b emit ultraviolet light having an intensity distribution in which the ultraviolet intensity is higher near the center of the straight pipe 220 and lower near the inner wall surface 220a of the straight pipe 220.

The inner diameter of the straight pipe 220 and the average flow velocity of a fluid flowing through the flow passage 112 are adjusted so that the fluid flows through the flow passage 212 in a laminar state. Accordingly, the flow velocity distribution is made so that the flow velocity of the fluid flowing near the central axis of the straight pipe 220 is relatively higher, and the flow velocity of the fluid flowing near the inner wall surface 220a of the straight pipe 220 is relatively lower. The fluid with such a velocity distribution is irradiated with ultraviolet light having an intensity distribution in which the ultraviolet intensity is higher near the center of the straight pipe 220 and lower near the inner wall surface 220a, emitted by the first light sources 240a and the second light sources 240b. Therefore, also in the present embodiment, a fluid in a laminar state is irradiated with ultraviolet light of which the intensity distribution corresponds to the flow velocity distribution of a fluid in a laminar state, so that the sterilization effect on the fluid can be improved.

Also, in the present embodiment, since the inlet port 223 and the outlet port 224 are disposed on the central axis of the straight pipe 220, generation of turbulence or a swirl in the fluid flowing through the flow passage 212 can be reduced. Further, since the light sources 240a and 240b are provided respectively at the inlet port 223 and the outlet port 224, the amount of ultraviolet light energy acting on the fluid can be increased compared to the case in which ultraviolet light is emitted from only one side, so that the sterilization effect on the fluid can be improved.

In a modification, the light sources may be provided at only one of the inlet port 223 and the outlet port 224. Also, the light sources 240a and 240b may be provided inside the straight pipe 220. When the light sources 240a and 240b are provided inside the straight pipe 220, the light sources 240a and 240b are respectively mounted on end surfaces 221a and 222b of the straight pipe 220, and cover members or the likes, which transmit ultraviolet light, are provided so that the light sources are not directly in contact with the fluid flowing within the flow passage 212.

The present invention has been described with reference to embodiments. It should be understood by those skilled in the art that the invention is not limited to the above-described embodiments and that various modifications could be developed on the basis of various design modifications and such modifications also fall within the scope of the present invention.

The fluid sterilization devices 10 according to the aforementioned embodiments are described as devices for performing sterilization treatment by irradiating a fluid with ultraviolet light. In a modification, the fluid sterilization devices may be used for purification treatment for decomposing an organic substance included in a fluid by irradiation of ultraviolet light.

In a modification, a straightening plate may be provided, midway along the flow passage constituted by a straight pipe, at the inlet port, or on the upstream side of the inlet port described above. The straightening plate may have a function to straighten the flow of a fluid flowing through the flow passage so as to make the flow a laminar flow. With the straightening plate, a laminar state with less turbulence can be formed, so that the sterilization effect can be improved.

In a modification, the light sources may have an adjustment mechanism for adjusting the intensity distribution of ultraviolet light emitted by the light emitting elements. The adjustment mechanism may include a transmitting optical element, such as a lens, and a reflecting optical element, such as a concave mirror. The adjustment mechanism may adjust the intensity distribution of ultraviolet light emitted by the light emitting elements so as to make the intensity distribution of ultraviolet light outputted by the light sources correspond to the velocity distribution of a fluid in a laminar state. With such an adjustment mechanism, a fluid can be irradiated with ultraviolet light of which the intensity distribution is suitable to the state of flow of the fluid, so that the sterilization effect can be further improved.

### [EXPLANATION OF REFERENCE NUMERALS]

- 10: fluid sterilization device
- 12: flow passage
- 20: straight pipe
- 21: first end part
- 22: second end part
- 23: inlet port
- 24: outlet port
- 40: light source
- 42: light emitting element
- 140: light source
- 210: fluid sterilization device
- 212: flow passage
- 220: straight pipe
- 221: first end part
- 222: second end part
- 223: inlet port
- 224: outlet port
- 240a: first light sources
- 240b: second light sources

### [INDUSTRIAL APPLICABILITY]

The present invention enables improvement in irradiation efficiency of ultraviolet light for a fluid flowing within a flow passage, thereby also improving the sterilization capability.

## Claims

1. A fluid sterilization device, comprising:
a straight pipe that constitutes a flow passage extending in a longitudinal direction; and
a light source that emits ultraviolet light in the longitudinal direction toward a fluid flowing in a laminar state within the flow passage, wherein
the light source includes a light emitting element for emitting ultraviolet light and emits ultraviolet light so as to provide an intensity distribution on a cross section of the flow passage perpendicular to the longitudinal direction, in which the ultraviolet intensity in an area near the center is higher than the ultraviolet intensity around the area.

2. The fluid sterilization device of claim 1, wherein:
the straight pipe includes a first end part provided with an inlet port through which a fluid flows into the straight pipe in the longitudinal direction, and also includes a second end part located opposite to the first end part; and
the light source is disposed at the second end part.

3. The fluid sterilization device of claim 2, wherein the light source and the inlet port are disposed on the central axis of the straight pipe.

4. The fluid sterilization device of claim 2 or 3, wherein the straight pipe includes an outlet port provided on the second end part, through which a fluid flows out in a direction intersecting the longitudinal direction.

5. The fluid sterilization device of claim 2, wherein:
the straight pipe includes an outlet port provided on the second end part, through which a fluid flows out in the longitudinal direction;
the light source includes a plurality of light emitting elements disposed so as to surround the outlet port; and
the plurality of light emitting elements emit ultraviolet light in the longitudinal direction toward a fluid flowing within the flow passage.

6. The fluid sterilization device of any one of claims 1 through 5, further comprising a straightening plate that makes a flow of a fluid within the flow passage a laminar flow.

7. A fluid sterilization method, in which a flow of a fluid in a laminar state is made within a straight pipe that constitutes a flow passage extending in a longitudinal direction, and ultraviolet light is emitted in the longitudinal direction toward a fluid flowing in a laminar state within the flow passage so as to provide an intensity distribution on a cross section of the flow passage perpendicular to the longitudinal direction, in which the ultraviolet intensity in an area near the center is higher than the ultraviolet intensity around the area.
